# FASCICULE DE BREVET EUROPEEN

(11) **EP 2 925 434 B1**
(45) Date de publication et mention de la délivrance du brevet: **24.11.2021**
(21) Numéro de dépôt: 13808119.5
(22) Date de dépôt: 27.11.2013
(51) Int. Cl.: B01J 23/26, B01J 23/00, B01J 37/04, B01J 37/26, C07C 17/20, B01J 37/00, B01J 23/86

(54) **CATALYSEUR PRÉPARÉ PAR BROYAGE RÉACTIF**
KATALYSATOR HERGESTELLT MITTELS REAKTIVMAHLEN
CATALYST PREPARED BY REACTIV MILLING

(30) Priorité: 03.12.2012 FR 1261536
(43) Date de publication de la demande: 07.10.2015
(73) Titulaire: Arkema France, 92700 Colombes (FR)
(72) Inventeur: DUBOIS, Jean-Luc, 69390 Millery (FR)
(74) Mandataire: Arkema Patent
(86) Numéro de dépôt international: PCT/FR2013/052872
(87) Numéro de publication internationale: WO 2014/087076

(56) Documents cités:
- EP-A1- 0 657 408
- WO-A1-00/03947
- CN-A- 101 385 977
- CN-A- 102 671 670
- FR-A1- 2 684 567
- FR-A1- 2 891 163
- US-A1- 2005 228 202
- US-B1- 6 433 233
- MARINKOVIC Z V ET AL: "Microstructural characterization of mechanically activated ZnO-Cr2O3 system", JOURNAL OF THE EUROPEAN CERAMIC SOCIETY, ELSEVIER SCIENCE PUBLISHERS, BARKING, ESSEX, GB, vol. 25, no. 12, 26 mai 2005 (2005-05-26), pages 2081-2084, XP027618400, ISSN: 0955-2219 [extrait le 2005-01-01]
- B.L. CHAMBERLAND, C.G.FREDERICK, J.L. GILLSON: "Synthesis and properties of several members of the series CrO(2-x)F(x)", JOURNAL OF SOLID STATE CHEMISTRY, vol. 6, no. 4, avril 1973 (1973-04), pages 561-564, XP002698400, DOI: 10.1016/s0022-4596(73)80015-5 cité dans la demande

## Description

### Domaine de l'invention.

La présente invention concerne la préparation d'un catalyseur, notamment à base d'oxyfluorure de chrome, par broyage réactif.

### Arrière plan technologique de l'invention.

Les oxydes de chrome peuvent être utilisés comme catalyseurs de réaction d'échange ou de réorganisation d'halogènes dans des composés hydrocarbonés ou d'hydrocarbures halogénés.

Les propriétés de ces catalyseurs peuvent être entre autres modulées par leurs méthodes de production et par leurs compositions.

Les catalyseurs à base d'oxydes de chrome sont généralement produits par précipitation de sels par des agents alcalins, suivie des étapes de lavage, séchage et calcination. A titre d'exemple, on peut citer le document, US 6,337,299 décrivant un procédé d'obtention de catalyseur de fluoration comprenant un oxyde de chrome produit par précipitation, suivie des étapes de séchage, compression sous forme de granules et calcination.

Les oxydes de chrome peuvent être produits sous différentes formes, cristallins ou amorphes.

Les catalyseurs de fluoration à base d'oxyde de chrome peuvent comprendre des dopants comme par exemple le nickel (FR 2 684 567), le vanadium, magnésium ou zinc (FR 2 713 633) ou l'indium, le gallium, le cobalt, le nickel, le zinc et l'aluminium (US 6,433,233).

La préparation des catalyseurs à base d'oxyde de chrome dopé s'effectue en général par co-précipitation. Après séchage et calcination, le solide est souvent soumis à une étape de traitement avec de l'HF gazeux ou d'un gaz fluoré, et/ou d'activation.

Le document FR 2 684 567 A1 décrit (page 3) la préparation de catalyseurs de fluoration par gélification, les produits utilisés pour préparer lesdits catalyseurs comprenant (page 4) un précurseur de chrome tel que l'un des oxydes de chrome et un précurseur de nickel tel que l'hydroxyde, l'un des oxyhalogénures, le nitrate ou le sulfate de nickel. Le document EP 0 657 408 A1 décrit (page 4, lignes 12-14) la préparation de catalyseurs de fluoration à base d'oxyde de chrome ou d'oxyde de chrome et d'au moins un autre métal catalytiquement actif, caractérisé en ce que la majeure partie du ou des oxydes est à l'état cristallisé. Différentes techniques connues sont mises en ouvre , par exemple la co-précipitation sous forme d'hydroxydes de différents sels métalliques ou la réduction de Crûs. La phase cristallisée est pour sa part préférentiellement choisie parmi les oxydes Cr₂O₃, CrO₂, NiCr₂O₄, NiCrO₃, NiCrO₄, MgCrO₄, ZnCr2O₄ ou un mélange de ces oxydes.

Les méthodes de préparation telles que décrites ci-dessus ne donnent pas entière satisfaction. En effet, la méthode de co-précipitation est limitée par le domaine de solubilité et est par conséquent peu flexible. Elle conduit aussi à une composition hétérogène et/ou des solides avec des surfaces spécifiques peu développées ce qui peut limiter les performances catalytiques. En outre, les méthodes précitées consomment d'importantes quantités d'eau et d'énergie.

La demanderesse a maintenant mis au point un procédé de préparation d'un solide à base de chrome ne présentant pas en partie ou en totalité les inconvénients des méthodes décrites dans l'art antérieur.

### Résumé de l'invention.

La présente invention concerne un procédé de préparation d'un catalyseur, comprenant le broyage réactif d'un premier réactif qui est un composé oxyde de chrome avec un deuxième réactif choisi parmi le CaCr₂O₇, le CaCrO₄, le Ca₃Cr₂O₈, le CaCrO₄, le CrVO₄, le Cr₂V₄O₁₃, le NiCr₂O₄, le NiCrO₄, le CuCrO₄, le CdCrF₅, le Pb₃Cr₂F₁₂, le Pb₄CrF₁₁, le Pb₅CrO₈, le Pb₂CrO₅, le PbCrO₄, le Pb₅CrF₁₇, le Pb₅W₃O9F₁₀, le CrF₂, le CrF₃, le CrOF₂, le CuF₂, le PbF₂, le BiF₃, le BiF₅, le CdF₂, le NiF₂, le YF₃, le MoF₃, le MoF₄, le MoF₅, le MoF₆, le MoOF₄, le GeF₂, le GeF₄, le SbF₃, le SbF₅, le BaF₂, le LaF₃, le LaOF, le PF₃, le SrF₂, le WF₄, le WF₅, le WF₆, le WOF₄, le ZnF₂, le MnF₃, le MnF₂, le SnF₂, le SnF₄, le CaF₂, le NbF₃ le NbF₄, le NbF₅, le NbO₂F, le MgF₂, le CeF₄, le TiF₃, le TiF₄, le TaFs, le ThF₄, le FeF₃, l'AIF₃, le ScF₃, le Mn₂O₃ substitué par du chrome, le Cr₂O₃ substitué par du manganèse, le MoO_{2,4}F_{0,3}, le Mo₄O_{11,2}F_{0,8} et les combinaisons de ceux-ci, caractérisé en ce qu'à l'issue de l'étape de broyage réactif, une étape de fluoration est mise en oeuvre par mise en contact avec un agent de fluoration.

Selon un mode de réalisation, le catalyseur comprend du chrome présentant un degré d'oxydation compris entre 3 et 5, de préférence de 3,1 à 4,5 et de manière plus particulièrement préférée de 3,2 à 4,2.

Selon un mode de réalisation, le premier réactif est choisi parmi le CrO₃, le Cr₂O₃, le CrO₂ et les combinaisons de ceux-ci.

Selon un mode de réalisation, l'étape de fluoration est mise en oeuvre de préférence par mise en contact avec

Selon un mode de réalisation, le broyage réactif est effectué pendant une durée de 1 à 96 heures, de préférence de 2 heures à 48 heures.

Selon un mode de réalisation, le deuxième réactif est fourni par un catalyseur usagé, ledit catalyseur usagé comprenant de préférence du chrome, de l'oxygène, du fluor, optionnellement un élément supplémentaire et / ou du coke ; ou le deuxième réactif est un fluorure de chrome, un oxyfluorure de chrome ou un oxyde de chrome dans lequel le degré d'oxydation du chrome est différent de celui du premier réactif.

### Exposé détaillé de l'invention.

L'invention est maintenant décrite plus en détail et de façon non limitative dans la description qui suit.

La présente invention a donc pour objet un procédé de préparation d'un catalyseur (notamment un oxyfluorure de chrome), comprenant le broyage réactif d'un premier réactif qui est un composé oxyde de chrome avec un deuxième réactif choisi parmi le CaCr₂O₇, le CaCrO₄, le Ca₃Cr₂O₈, le CaCrO₄, le CrVO₄, le Cr2V₄O₁₃, le NiCr2O4, le NiCrO₄, le CuCrO₄, le CdCrF₅, le Pb₃Cr₂F₁₂, le Pb₄CrF₁₁, le Pb₅CrO₈, le Pb₂CrO₅, le PbCrO₄, le Pb₅CrF₁₇, le Pb₅W₃O₉F₁₀, le CrF₂, le CrF₃, le CrOF₂, le CuF₂, le PbF₂, le BiF₃, le BiF₅, le CdF₂, le NiF₂, le YF₃, le MoF₃, le MoF₄, le MoF₅, le MoF₆, le MoOF₄, le GeF₂, le GeF₄, le SbF₃, le SbF₅, le BaF₂, le LaF₃, le LaOF, le PF₃, le SrF₂, le WF₄, le WF₅, le WF₆, le WOF₄, le ZnF₂, le MnF₃, le MnF₂, le SnF₂, le SnF₄, le CaF₂, le NbF₃, le NbF4, le NbF5, le NbO₂F, le MgF₂, le CeF₄, le TiF₃, le TiF₄, le TaF₅, le ThF₄, le FeF₃, l'AIF₃, le ScF₃, le Mn₂O₃ substitué par du chrome, le Cr₂O₃ substitué par du manganèse, le MoO_{2,4}F_{0,3}, le Mo₄O_{11,2}F_{0,8} et les combinaisons de ceux-ci, caractérisé en ce qu'à l'issue de l'étape de broyage réactif, une étape de fluoration est mise en oeuvre par mise en contact avec un agent de fluoration.

Le cadmium, le gallium, le germanium, l'indium, le scandium et le thorium sont des éléments présentant des problèmes de sécurité ou de rareté, aussi il est préféré que les éléments autres que l'oxygène et le fluor soient choisis parmi la liste restreinte comprenant le chrome, le nickel, le magnésium, le cobalt, le zinc, l'aluminium, l'antimoine, le baryum, le bismuth, le calcium, le cérium, le cuivre, l'étain, le fer, le lanthane, le manganèse, le molybdène, le niobium, le phosphore, le plomb, le strontium, le tantale, le terbium, le titane, le tungstène, le vanadium, l'yttrium et le zirconium.

Le deuxième réactif peut être généré *in-situ* à partir d'un précurseur (notamment sous forme de sel ne donnant pas de résidu solide après calcination tel que carbonate, nitrate, hydroxyde, oxyhydroxyde, ammonium).

le deuxième réactif comprend deux éléments et l'un de ces éléments est du chrome. Selon un mode de réalisation alternatif, ces deux éléments sont distincts du chrome.

Le deuxième réactif comprend notamment deux éléments, lorsque le deuxième réactif est fourni par un catalyseur usagé (par exemple un catalyseur à base d'oxyfluorure de chrome dopé au nickel, au zinc...). Le catalyseur usagé peut être soumis à un traitement préalable par exemple un décokage, ou un lessivage afin d'éliminer d'éventuels contaminants tels que Na, Si, Fe.

Dans le catalyseur obtenu (qui est de préférence un oxyfluorure de chrome comprenant éventuellement au moins un autre élément métallique), le degré d'oxydation du chrome est supérieur à 3 et inférieur à 5, avantageusement inférieur ou égal à 4,5 et mieux encore inférieur ou égal à 4,2.

Le degré d'oxydation du chrome dans l'oxyfluorure particulièrement préféré est supérieur ou égal à 3,1 ou mieux encore supérieur ou égal à 3,2.

Le deuxième réactif peut par exemple être un oxyde, un fluorure ou un oxyfluorure mixte choisi parmi CaCr₂O₇, CaCrO₄, Ca₃Cr₂O₈, CaCrO₄, CrVO₄, Cr₂V₄O₁₃, MoOF₄, NiCr₂O₄, NiCrO₄, CuCrO₄, CdCrF₅, Pb₃Cr₂F₁₂, Pb₄CrF₁₁, Pb₅CrO₈, Pb₂CrO₅, PbCrO₄, Pb₅CrF₁₇, WOF₄ et Pb₅W₃O₉F₁₀.

L'oxyde mixte peut également être une solution solide dans lequel Mn est substitué par le chrome dans Mn₂O₃, ou le chrome est substitué par Mn dans Cr₂O₃.

Des solutions solides de chrome dans NiO du type NiCrₐO₄ avec a compris entre 1 et 2 peuvent également convenir.

Selon un mode de réalisation, le deuxième réactif est un fluorure simple (avec x égal à zéro), par exemple choisi parmi CrF₂, CrF₃, CuF₂, PbF₂, BiF₃, BiF5, CdF₂, NiF₂, YF₃, MoF₃, MoF₄, MoF₅, MoF₆, GeF₂, GeF₄, SbF₃, SbF₅, BaF₂, LaF₃, PF₃, SrF₂, WF₄, WF₅, WF₆, ZnF₂, MnF₃, MnF₂, SnF₂, SnF₄, CaF₂, NbF₃ NbF₄ NbF₅, MgF₂, TiF₃, TiF₄, TaF₅, ThF₄, FeF₃, AlF₃, ScF₃, CeF₄.

Selon un mode de réalisation, le deuxième réactif est un oxyfluorure simple tel que MoO_{2,4}F_{0,6} et Mo₄O_{11,2}F_{0,8}, NbO₂F, CrOF₂, ou par exemple un catalyseur usagé comprenant du chrome, de l'oxygène et du fluor, éventuellement un élément supplémentaire autre que le chrome, l'oxgène et le fluor et du coke.

Le degré d'oxydation du chrome dans le premier réactif oxyde de chrome peut valoir de 3 à 6. Cet oxyde de chrome peut être notamment choisi parmi le CrO₃, le Cr₂O₃ et le CrO₂. Il peut également être généré *in situ* à partir d'un précurseur tel qu'un nitrate, un carbonate, un hydroxyde, un ammonium ou un oxyhydroxyde de chrome.

Le deuxième réactif peut être un catalyseur usagé, ou encore un oxyde de chrome dans lequel le chrome a un degré d'oxydation différent de celui du premier réactif. Notamment, lorsque le degré d'oxydation du chrome dans le premier réactif est égal à 3, au moins l'un parmi les éléments du deuxièrem réactif représente un élément de degré d'oxydation supérieur à 3.

Lorsque le deuxième réactif est un oxyde de chrome, un fluorure de chrome ou un oxyfluorure de chrome, le rapport de la quantité molaire du deuxième composé par rapport à celle du premier composé qui est mise en jeu dans l'étape de broyage réactif dépend du degré d'oxydation respectif du chrome dans les deux réactifs respectifs. Ce rapport molaire est noté R. En notant CrₐO_{b} le premier réactif oxyde de chrome, et compte tenu du fait que l'on souhaite que le produit solide soit fluoré d'au moins 10 % et d'au plus 90 % par exemple (une fluoration excessive le rendant inerte), le nombre 2y/((x+R·b)+2y) vaut de préférence d'environ 0,1 à environ 0,9, autrement dit que le rapport R vaut de préférence environ de (1/b)·(0,2y/0,9-x) à (1/b)·(18y-x).

Lorsque le deuxième réactif ne comprend pas de chrome, le rapport molaire R représentant la quantité molaire du deuxième réactif par rapport à celle du premier réactif (exprimé en quantité de M et M' par rapport à la quantité de chrome) qui est mise en jeu dans l'étape de broyage réactif est supérieure à 0 et de préférence inférieure ou égale à 8,5 et avantageusement inférieure ou égale à 1,5.

L'oxyfluorure de chrome final peut ne contenir que du chrome, de l'oxygène et du fluor, auquel cas le deuxième réactif est de préférence le CrF₃ ou CrOF₂ ou CrO₂F₂ ou CrF₂. CrF₃ et CrF₂ peuvent être générés in-situ à partir du (NH₄)₃CrF₆.

Alternativement, l'oxyfluorure de chrome peut contenir un dopant, auquel cas le deuxième réactif est avantageusement choisi parmi l'oxyde de zinc, l'oxyde de nickel, le fluorure de nickel et l'oxyde de manganèse.

Il est possible d'utiliser une combinaison d'oxydes de chrome différents au titre du premier réactif, et / ou d'utiliser une combinaison de différents composés de formule M_{z}M'_{1-z}OₓF_{y} au titre du deuxième réactif.

La présente invention met en oeuvre un broyage réactif, qui relève de la mécano-chimie. La mécano-chimie concerne les réactions chimiques des matériaux induites directement par l'absorption d'énergie mécanique. Les méthodes de cisaillement, de frottement et de broyage peuvent impliquer des phénomènes mécano-chimiques. Ces phénomènes abrasifs peuvent non seulement induire l'augmentation de la surface effective par la diminution de la taille des particules mais peuvent aussi former de nouveaux alliages par mécano-chimie.

Selon Denis et al. (J. Appl. Electrochem., Volume 29, n°8, 1999, p.951-960), des alliages peuvent être produits par diffusion des éléments à l'état solide, favorisée par la déviation de la structure cristalline après déformation mécanique. Dans le cas du broyage, la température peut croître de quelques dizaines de degré sans dépasser localement 300°C.

Dans ce qui précède et ce qui suit, on entend par broyage réactif un procédé mécanique de réduction de taille des particules de solide induisant simultanément une réaction chimique et une modification de la composition des particules de solide, s'accompagnant d'une agglomération des particules.

De préférence, le broyage réactif est effectué en utilisant une énergie de 1 à 10000 Wh/kg, de manière plus particulièrement préférée de 10 à 1000 Wh/kg.

L'énergie mise en oeuvre au cours du broyage dépend de l'équipement de broyage et de la durée du broyage. Ainsi pour amener un solide à des dimensions micrométriques, une énergie de 50 Wh/kg est typiquement utilisée. Un broyeur à boulet nécessiterait une durée de 1 heure à 50 W/kg.

L'étape de broyage réactif peut être mise en oeuvre dans une large gamme d'équipements de broyage ou de concassage. On peut citer notamment les broyeurs contenant des éléments mobiles tels que des boulles, des cylindres, des barres ou même contenant uniquement les particules à broyer qui se broient de manière autonome par collisions. Le broyage peut être effectué par tous moyens connu de l'homme du métier, par exemple par force centrifuge ou par oscillation ou bien par une combinaison des deux.

L'étape de broyage réactif peut être effectuée de manière continue, semi-continue, ou discontinue. Le broyage peut être effectué soit à sec soit en suspension lorsque le deuxième réactif est un liquide.

Ainsi, le broyage réactif diffère de la méthode utilisée par Chamberland et al., dans Journal of solid state chemistry 6, 561-564 (1973), en ce que, dans cet article, des mélanges de CrO₂ et CrF₂ sont broyés dans un mortier puis placés dans une capsule pour être soumis à une pression de 60-65 kbar et 1200°C pendant 2 heures, si bien que la réaction entre les deux espèces n'est pas réalisée par le broyage mais par l'étape ultérieure de chauffage.

Les avantages du procédé selon la présente invention sont les suivants :
- flexibilité : la composition du solide peut être facilement modulée avec la même installation de production ;
- économie : le procédé est facilement industrialisable ;
- sécurité : il n'y a pas de déchets ni de rejets aqueux ;
- recyclabilité : le procédé permet le recyclage des solides (catalyseurs) usagés à condition qu'ils ne soient pas excessivement pollués par des métaux ;
- possibilité d'obtention d'agrégats de taille micrométrique voire millimétrique à partir des particules nanométriques.

Le broyage réactif se distingue du simple broyage par une modification structurelle et texturale du ou des solides.

On ajuste de préférence les paramètres du broyage réactif de telle sorte que les particules broyées s'agglomèrent jusqu'à obtenir une distribution de particules ayant un Dv50 compris entre 20 et 100 µm, et plus particulièrement entre 40 et 80 µm. Une telle distribution de taille permet d'obtenir un produit solide en poudre facilement manipulable et fluidisable.

Le procédé selon l'invention comprend une étape de fluoration, à la suite de l'étape de broyage réactif.

De préférence, l'agent de fluoration est l'acide fluorhydrique anhydre.

L'étape de fluoration selon le procédé de la présente invention peut être mise en oeuvre à une température comprise entre 100 et 450°C, de préférence entre 200 et 350°C pour une durée comprise entre 1 et 50 heures.

### PARTIE EXPERIMENTALE

On utilise un broyeur planétaire RETSCH, modèle PM400MA, avec un rapport de vitesse de rotation bol/roue solaire de 1/-3. On utilise des bols de 125 ml. Le broyage est effectué à l'air, pour des durées de 60 minutes, avec une vitesse de rotation de la roue solaire de 400 tr/min. On utilise des billes en zircone de 10 mm de diamètre, soit 30 billes. Le volume d'échantillon est de 15 à 50 ml.

On utilise de l'oxyde de chrome Cr₂O₃ de Sigma-Aldrich, de granulométrie d'environ 50 microns. (ref catalogue 393703-500G) de masse molaire 151,99 g/mole ; de l'oxyde de chrome CrO₃ de Sigma-Aldrich (ref catalogue 236470-500G) de masse molaire 99,99 g/mole et de surface spécifique de 5 m²/g; du nitrate de Chrome nona hydrate (Cr(NO₃)₃, 9H₂O) de Sigma-Aldrich (référence catalogue 239259-500G), masse molaire 400,15 g/mole.

On utilise aussi de l'oxyde de Nickel (NiO) de Sigma-Aldrich (ref catalogue 399523) de moins de 50 microns, et de masse molaire 74,69 g/mole; de l'oxyde de terbium (Tb₄O₇) (ref catalogue 253952-10G) de masse molaire 747,7 g/mole; de l'oxyde de zinc (ZnO) (référence catalogue 205532-100g) de masse molaire 81,39 g/mole.

On utilise du fluorure de chrome fraîchement préparé comme suit : on prépare une solution aqueuse de nitrate de chrome en mélangeant 400 g de nitrate de chrome (1 mole) dans 7 litres d'eau déminéralisée, et on ajoute ensuite environ 1 kg d'une solution d'ammoniaque à 10 % poids pour provoquer la précipitation d'un hydroxyde de chrome. Le précipité est filtré et lavé à l'eau, jusqu'à ce que les eaux de lavage aient un pH stable. Le précipité est séché sous air à 120 °C dans une étuve, pendant une nuit, et concassé et broyé pour obtenir une poudre de moins de 50 microns. La poudre ainsi obtenue est mélangée avec 2 % poids de graphite pour permettre un pastillage sous forme de cylindres de 5 mm de diamètre et 5 mm de hauteur, avec un trou central de 2,5 mm de diamètre intérieur. Les pastilles sont chargées dans un tube en hastelloy de 21 mm de diamètre intérieur, puis calcinées à 400 °C, pendant 2 heures sous un flux d'azote. Ensuite les pastilles sont traitées par un flux de fluorure d'hydrogène en mélange avec de l'azote, jusqu'à une température de 360 °C.

Le solide obtenu est caractérisé par une surface spécifique de 41 m²/g et il est amorphe aux rayons X.

### Exemple 1

On mélange 76 g de Cr₂O₃, 4 g de ZnO, et 18 g de NiO. Le mélange a une surface spécifique avant broyage de 4,5 m²/g.

Après broyage réactif d'une heure dans les conditions décrites ci-dessus le mélange a une surface spécifique (BET) de 8.3 m²/g

### Exemple 2

On mélange 54 g de flurorure de Chrome, 25 g de CrO3. Le mélange a une surface spécifique avant broyage de 15,2 m²/g.

Après broyage réactif d'une heure dans les conditions décrites ci-dessus le mélange a une surface spécifique (BET) de 26 m²/g

### Exemple 3

On mélange 76 g de Cr₂O₃, et 15 de NiO et 5 g de Tb₄O₇. Le mélange a une surface spécifique avant broyage de 4,7 m²/g.

Après broyage réactif d'une heure dans les conditions décrites ci-dessus le mélange a une surface spécifique (BET) de 7,6 m²/g

### Exemple 4

On mélange 100 g de l'hydroxyde de chrome obtenu lors de la préparation du fluorure de Chrome, et 50 g de CrO₃.

L'efficacité du broyage réactif est aussi suivi par analyse DRX (Source Cu, Kα) des mélanges. On analyse chaque mélange avant et après broyage, et on caractérise l'efficacité du broyage par la disparition et l'élargissement des raies de diffraction X. Les produits obtenus sont caractérisés par des pics larges traduisant le caractère plus amorphe des solides formés.

Dans l'exemple 1, la raie de diffraction X à 33,6 ° (caractéristique de Cr₂O₃) a une intensité relative de 20 % de celle du mélange de départ. La raie de diffraction X à 43,3 ° (caractéristique de NiO) a une intensité relative de 5 % de celle du mélange de départ.

Dans l'exemple 2, la modification du mélange de départ au cours du broyage réactif est illustrée par l'apparition d'une raie à 28,5 °.

Dans l'exemple 3, la raie de diffraction X à 33,6 ° a une intensité relative de 27 % de celle du mélange de départ.

Dans l'exemple 4, une raie large de diffraction à environ 28,5° est observée.

## Revendications

1. Procédé de préparation d'un catalyseur de fluoration, comprenant le broyage réactif d'un premier réactif qui est un composé oxyde de chrome avec un deuxième réactif choisi parmi le CaCr₂O₇, le CaCrO₄, le Ca₃Cr₂O₈, le CaCrO₄, le CrVO₄, le Cr₂V₄O₁₃, le NiCr₂O₄, le NiCrO₄, le CuCrO₄, le CdCrF₅, le Pb₃Cr₂F₁₂, le Pb₄CrF₁₁, le Pb₅CrO₈, le Pb₂CrO₅, le PbCrO₄, le Pb₅CrF₁₇, le Pb₅W₃O₉F₁₀, le CrF₂, le CrF₃, le CrOF₂, le CuF₂, le PbF₂, le BiF₃, le BiF₅, le CdF₂, le NiF₂, le YF₃, le MoF₃, le MoF₄, le MoF₅, le MoF₆, le MoOF₄, le GeF₂, le GeF₄, le SbF₃, le SbF₅, le BaF₂, le LaF₃, le LaOF, le PF₃, le SrF₂, le WF₄, le WF₅, le WF₆, le WOF₄, le ZnF₂, le MnF₃, le MnF₂, le SnF₂, le SnF₄, le CaF₂, le NbF₃ le NbF₄, le NbF₅, le NbO₂F, le MgF₂, le CeF₄, le TiF₃, le TiF₄, le TaF₅, le ThF₄, le FeF₃, l'AIF₃, le ScF₃, le Mn₂O₃ substitué par du chrome, le Cr₂O₃ substitué par du manganèse, le MoO_{2,4}F_{0,6}, le Mo₄O_{11,2}F_{0,8} et les combinaisons de ceux-ci, **caractérisé en ce qu'**à l'issue de l'étape de broyage réactif, une étape de fluoration est mise en œuvre par mise en contact avec un agent de fluoration.

2. Procédé selon la revendication 1, dans lequel le catalyseur comprend du chrome présentant un degré d'oxydation compris entre 3 et 5, de préférence de 3,1 à 4,5 et de manière plus particulièrement préférée de 3,2 à 4,2.

3. Procédé selon la revendication 1 ou 2, dans lequel le premier réactif est choisi parmi le CrO₃, le Cr₂O₃, le CrO₂ et les combinaisons de ceux-ci.

4. Procédé selon l'une des revendications 1 à 3, comprenant, à l'issue de l'étape de broyage réactif, une étape de fluoration par mise en contact avec de l'acide fluorhydrique anhydre.

5. Procédé selon l'une des revendications 1 à 4, dans lequel le broyage réactif est effectué pendant une durée de 1 à 96 heures, de préférence de 2 heures à 48 heures.

6. Procédé selon l'une des revendications 1 à 5, dans lequel le deuxième réactif est fourni par un catalyseur usagé, ledit catalyseur usagé comprenant de préférence du chrome, de l'oxygène, du fluor, optionnellement un élément supplémentaire et / ou du coke ; ou dans lequel le deuxième réactif est un fluorure de chrome, un oxyfluorure de chrome ou un oxyde de chrome dans lequel le degré d'oxydation du chrome est différent de celui du premier réactif.

## Patentansprüche

1. Verfahren zur Herstellung eines Fluorierungskatalysators, umfassend das Reaktivmahlen eines ersten Reagens, bei dem es sich um eine Chromoxidverbindung handelt, mit einem zweiten Reagens, das aus CaCr₂O₇, CaCrO₄, Ca₃Cr₂O₈, CaCrO₄, CrVO₄, Cr₂V₄O₁₃, NiCr₂O₄, NiCrO₄, CuCrO₄, CdCrF₅, Pb₃Cr₂F₁₂, Pb₄CrF₁₁, Pb₅CrO₈, Pb₂CrO₅, PbCrO₄, Pb₅CrF₁₇, Pb₅W₃O₉F₁₀, CrF₂, CrF₃, CrOF₂, CuF₂, PbF₂, BiF₃, BiF₅, CdF₂, NiF₂, YF₃, MoF₃, MoF₄, MoF₅, MoF₆, MoOF₄, GeF₂, GeF₄, SbF₃, SbF₅, BaF₂, LaF₃, LaOF, PF₃, SrF₂, WF₄, WF₅, WF₆, WOF₄, ZnF₂, MnF₃, MnF₂, SnF₂, SnF₄, CaF₂, NbF₃, NbF₄, NbF₅, NbO₂F, MgF₂, CeF₄, TiF₃, TiF₄, TaF₅, ThF₄, FeF₃, AlF₃, ScF₃, chromsubstituiertem Mn₂O₃, mangansubstituiertem Cr₂O₃, MoO_{2,4}F_{0,6}, Mo₄O_{11,2}F_{0,8} und Kombinationen davon ausgewählt wird, **dadurch gekennzeichnet, dass** am Ende des Reaktivmahlschritts ein Fluorierungsschritt durch Inkontaktbringen mit einem Fluorierungsmittel durchgeführt wird.

2. Verfahren nach Anspruch 1, wobei der Katalysator Chrom in einer Oxidationsstufe zwischen 3 und 5, vorzugsweise von 3,1 bis 4,5 und weiter bevorzugt von 3,2 bis 4,2 umfasst.

3. Verfahren nach Anspruch 1 oder 2, wobei das erste Reagens aus CrO₃, Cr₂O₃, CrO₂ und Kombinationen davon ausgewählt wird.

4. Verfahren nach einem der Ansprüche 1 bis 3, das am Ende des Reaktivmahlschritts einen Fluorierungsschritt durch Inkontaktbringen mit wasserfreiem Fluorwasserstoff umfasst.

5. Verfahren nach einem der Ansprüche 1 bis 4, wobei das Reaktivmahlen über einen Zeitraum von 1 bis 96 Stunden, vorzugsweise von 2 Stunden bis 48 Stunden, durchgeführt wird.

6. Verfahren nach einem der Ansprüche 1 bis 5, wobei das zweite Reagens durch einen verbrauchten Katalysator bereitgestellt wird, wobei der verbrauchte Katalysator vorzugsweise Chrom, Sauerstoff, Fluor, gegebenenfalls ein zusätzliches Element und/oder Koks umfasst; oder wobei es sich bei dem zweiten Reagens um ein Chromfluorid, ein Chromoxidfluorid oder ein Chromoxid, in dem die Oxidationsstufe des Chroms von derjenigen des ersten Reagens verschieden ist, handelt.

## Claims

1. Process for preparing a fluorination catalyst, comprising the reactive milling of a first reagent, which is a chromium oxide compound, with a second reagent chosen from CaCr₂O₇, CaCrO₄, Ca₃Cr₂O₈, CaCrO₄, CrVO₄, Cr₂V₄O₁₃, NiCr₂O₄, NiCrO₄, CuCrO₄, CdCrF₅, Pb₃Cr₂F₁₂, Pb₄CrF₁₁, Pb₅CrO₈, Pb₂CrO₅, PbCrO₄, Pb₅CrF₁₇, Pb₅W₃O₉F₁₀, CrF₂, CrF₃, CrOF₂, CuF₂, PbF₂, BiF₃, BiF₅, CdF₂, NiF₂, YF₃, MoF₃, MoF₄, MoF₅, MoF₆, MoOF₄, GeF₂, GeF₄, SbF₃, SbF₅, BaF₂, LaF₃, LaOF, PF₃, SrF₂, WF₄, WF₅, WF₆, WOF₄, ZnF₂, MnF₃, MnF₂, SnF₂, SnF₄, CaF₂, NbF₃, NbF₄, NbF₅, NbO₂F, MgF₂, CeF₄, TiF₃, TiF₄, TaF₅, ThF₄, FeF₃, AlF₃, ScF₃, chromium-substituted Mn₂O₃, manganese-substituted Cr₂O₃, MoO_{2.4}F_{0.6}, Mo₄O_{11.2}F_{0.8} and combinations thereof, **characterized in that** at the end of the reactive milling step, a step of fluorination is carried out by bringing into contact with a fluorinating agent.

2. Process according to Claim 1, in which the catalyst comprises chromium having an oxidation state of between 3 and 5, preferably from 3.1 to 4.5 and more particularly preferably from 3.2 to 4.2.

3. Process according to Claim 1 or 2, in which the first reagent is chosen from CrO₃, Cr₂O₃, CrO₂ and combinations thereof.

4. Process according to one of Claims 1 to 3, comprising, at the end of the reactive milling step, a step of fluorination by bringing into contact with anhydrous hydrofluoric acid.

5. Process according to one of Claims 1 to 4, in which the reactive milling is carried out for a period of from 1 to 96 hours, preferably from 2 hours to 48 hours.

6. Process according to one of Claims 1 to 5, in which the second reagent is provided by a spent catalyst, said spent catalyst preferably comprising chromium, oxygen, fluorine, optionally an additional element and/or coke, or in which the second reagent is a chromium fluoride, a chromium oxyfluoride or a chromium oxide in which the oxidation state of the chromium is other than that of the first reagent.
